# FASCICULE DE BREVET EUROPEEN

(11) **EP 2 478 900 B1**
(45) Date de publication et mention de la délivrance du brevet: **13.07.2016**
(21) Numéro de dépôt: 11290534.4
(22) Date de dépôt: 24.11.2011
(51) Int. Cl.: A61K 31/198, A61K 9/14, A61P 15/08

(54) **Créatines monohydrate destinées a régulariser les sécrétions hormonales et l'infertilité durant le cycle, la fécondation in vitro, et l'insémination artificielle**
Kreatin-Monohydrat zur Regulierung der Hormonabscheidungen und der Unfruchtbarkeit während des Zyklus, der In-vitro-Befruchtung und der künstlichen Besamung
Monohydrate creatins intended for regulating the hormone secretions and infertility during the cycle, the in vitro fertilisation and the artificial insemination

(30) Priorité: 21.01.2011 FR 1100192
(43) Date de publication de la demande: 25.07.2012
(73) Titulaire: Letourneur, Bernard, 72230 Guécélard (FR)
(72) Inventeur: Letourneur, Bernard, 72230 Guécélard (FR)

(56) Documents cités:
- WO-A1-01/70238
- DE-A1- 19 929 995
- DE-A1-102007 053 369
- GB-A- 1 603 639
- US-B1- 7 226 947

## Description

La composition a pour objet les créatines monohydrate ou citrate pur sous forme de poudre sels, micronisés destinée a régularisé les sécrétions hormonales,
et la stérilité de la femme.

La composition a également pour objet les créatines monohydrate , sels, destinée a augmenté le taux d oestradiol, croissance follicule,

La composition, a également pour objet les créatines monohydrate destinée a augmenté le taux d ovulation,
La composition, a également pour objet les créatines monohydrate destinée a augmenté le taux de progestérone, température thermique.
La composition, a également pour objet les créatines monohydrate pur permettant de régulariser les règles chez la femme et les animaux femelles et de favoriser 1 ovulation thermique ;

La composition comprend a titre d éléments actifs ,ingrédients créatines monohydrate ou citrate , sels sous forme de poudre selon une réalisation préférée de la découverte ; on administre les créatines monohydrate pure par voie orale
On peut également prévoir une administration rectale,

Lorsqu elle est administrée par voie orale , les créatines Peut comporté un excipients ou véhicule de préférence en poudre ou ne Pas comporter d excipient et être conditionnées dans des gélules,
Lorsqu elle comporte des excipients, la composition de les créatines monohydrate pure peut être conditionnée sous forme de pillules, de comprimés, de dragées , et sous toute autre forme appropriées. Capsules molles ;
Les créatines monohydrate ou citrate,sels, peut contenir en autre tout excipient
Ou véhicule couramment utilisé pour la préparation des médicaments.

On peut citer notamment :
Des véhicules tels que 1 amidon les sucres, le mannitol ; le lactose
   - des agents liants tels que le carboxycellulose, le carboxyméthylcellulose et autres dérivés cellulosiques, les alginates, la gélatine et la polyvinypyrrolidone ; les lubrifiants tels que le talc, les parfums et des agents améliorant le goût,

Des formes galéniques particulièrement avantageuses pour la voie orale sont les gélules, les comprimés, les dragées, mais 1 on peut administrer les créatines monohydrate pur sous toute forme galénique destinée à la voie orale. des excipients tels que le beurre de cacao, les huiles hydrogénées , les huiles hydrogénées et polyoxyéthylénées, les glycérides smi-synthétiques solides, Les polyoxyéthylèneglycols ainsi que les autres excipients habituellement Employés pour la confection des suppositoires.

La dose journalière recommandée est de 300mg la cure trois cycles Renfermant 300mg des créatines monohydrate ,sels soit5mg/kg :excipients La posologie recommandée a administré la dose ci-dessus Sous forme de gélules, ou comprimé, ou sous forme d ampoule, ou sous Forme de sirop, ; le soir aux repas ou le matin ; ou sous forme de suppositoires

Par voie rectale on contrôle 1 action des créatines monohydrate ,sels en effectuant le dosage hormonales par prise de sang d oestradiol, progestérone, aux 15ém et aux 24èm jours des règles

La Créatines monohydrate ou citrate en poudre ,sels selon sa composition de nombreuses, Femmes pendant des années ne pouvant pas être enceinte, et qui on pris la créatines monohydrate ,sels, son enceinte ; C est un progrès pour la médecine génétique, pour 1 ensemble de la Fertilité chez les femmes ,et les animaux femelles.

Les exemples ci-après de la découverte

### Exemple 1

Les femmes en insuffisance d oestradiol aux 15jours des règles a 10ng/ml ne secrètes pas de follicule ; pas ovocytes avec les créatines monohydrate le taux d oestradiol a augmenté en réalisant une mesure d analyse du laboratoire aux 15 em jours des règles on obtient un résultat de 400 ng/ml, donc des follicules,

### Exemple 2

Les femmes en insuffisance de progestérone du 15ém jours des règles Aux 28ém jours des règles donc la prise de sang est de 4 ng/ml Pas d ovulation ,la courbe de température est au-dessous de 36.8, Avec les créatines monohydrate ,sels, le taux de progestérone a augmenté En réalisant une mesure d analyse du laboratoire aux 28ém jours des Règles on obtient un résultat de 18ng/ml donc une ovulation Thermique aux dessus de 37.2

Cycle thermique avec les créatines monohydrate active

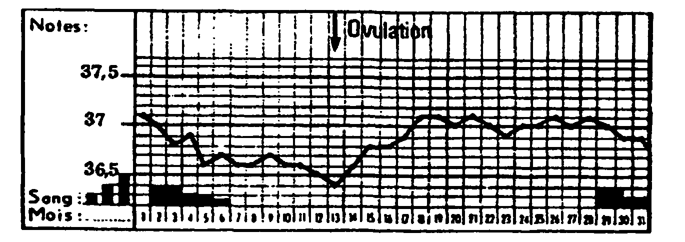

### Exemple 3

le taux d échec en fécondation in -vitro est énorme a cause de la réimplantation sur une courbe de température au -dessous de 36.8 donc pas d ovulation les créatines monohydrate ,sels, augmente le taux de réussite la progestérone augmente la courbe de température, donc ovulation thermique ,

### Exemple 4

pour 1 insémination artificielle chez la femme voir 1 exemple 1et2 la cure des créatines monohydrate ,sels est de 3 mois donc 3 cycles menstruels de la femme,vérifier les créatines prises de sang

## Revendications

1. Composition créatines monohydrate ou citrate ou une de ses sels ,sous forme de poudre micronisée pour une utilisation destinée à lutter contre la stérilité chez la femme et chez les animaux femelles - comprenant à titre d'éléments actifs et de traçabilité des créatines monohydrate, soit seul, soit en présence d' un excipient.

2. Composition pour son utilisation selon la revendication 1, **caractérisée par le fait que** le rapport en poids est de 300mg soit 5mg / kg.

3. Composition pour son utilisation selon l'une quelconque des revendications 1 à 2 - **caractérisée par le fait qu'**elle contient un excipient solide, et se présente sous forme de comprimés, de pilules ,de gélules, ou sous forme d'ampoules ou de sirop , ou toute autre forme solide.

4. Composition pour son utilisation selon l'une quelconque des revendications 1 à 3 -**caractérisée par le fait qu'**elle renferme également un adjuvant choisi parmi les véhicules, les agents, liants,les lubrifiants, les parfums et les agents améliorants le gout.

5. Composition pour son utilisation selon l'une quelconque des revendications 1 à 4- **caractérisée par le fait qu'**elle ne contient pas d'excipient et se présente sous forme de gélules.

6. Composition pour son utilisation selon l'une quelconque des revendications 1à5- **caractérisée par le fait qu'**elle contient des excipients et se présente sous forme d'ampoules ou de sirop.

7. composition pour son utilisation selon l'une quelconque 1à3-**caractérisée par le fait qu'**elle se présente sous forme de suppositoire.

## Patentansprüche

1. Zusammensetzung kreatin Monohydrat und citrat oder ein salz davon , in form von mikronisierten pulvers zur verwendung bei der bekampfung von unfruchtbarkeit bei frauen und weibliche tiere bestimmt -auch im wege der vermogenswerte und ruckverfolgbarkeit von kreatin monohydrat, entweder allein oder in der gegenwart eines hilfsstoffs.

2. Zusammensetzung zur verwendung nach anspruch 1 **dadurch gekennzeichnet dass** das gewichtsverhaltnis von 300mg oder 5mg/kg.

3. Zusammensetzung zur verwendung nach einem der anspruch 1 bis 2 **dadurch gekennzeichnet, dass** es einen festen trager enthalt und in form von tabletten, pillen , kapseln , oder in form von ampullen oder sirup oder je de andere feste form.

4. Zusammensetzung zur verwendung nach eimen der anspruch 1 bis 3-caracterisee gekennzeichnet, dab es auberdem ein aus fahrzeugen , bindemittel, gleitmittel, parfums ,adjuvans und geschmacksverbessernde mittel.

5. Zusammensetzung zur verwendung nach eimen der anspruch 1 bis 4-**dadurch gekennzeichnet, dass** sie keine exzipienten enthalten ist und in kapselform.

6. Zusammensetzung zur verwendung nach eimen der anspruch 1 bis 5- **dadurch gekennzeichnet**, dab si hilfsstoffe verwendet und in form von ampoullen oder sirup.

7. Zusammensetzung zurverwendung nach eimen 1a3-**dadurch gekennzeichnet** dab es sals suppositorum vorgeshen ist.

## Claims

1. Composition créatines monohydrate or a salt there of in the form of micronized power intended for use in the fight against infertility in women and female animals -including by way of assets and traceability of créatines monohydrate, either alone or in the presence of an excipient.

2. Composition for use according to claim 1, **characterized in that** the ratio by weight is 300mg or 5mg /kg.

3. Composition for use according to any one of claim 1 to 2-**characterized in that** it contains a solid excipient and is in the form of tablets , pills , capsules, or in the form of ampoules or syrup or any other solid form.

4. Composition for use according to any one of claim 1 to 3-**characterized in that** it also contains an adjuvant chosen from vehicles, agents, binders ,lubricants, perfumes and taste improving agents.

5. Composition for use according to any one of claim 1to4-**characterized in that** it does not contain any excipients and is in capsule form.

6. Composition for use according to any one of claim 1 to 5-charactérized in that it contains excipients and is in the form of ampoules or syrup.

7. Composition for use according to any one 1 to 3-charactérized in that it is provided as a suppository.
